(19) Europäisches Patentamt
European Patent Office
Office européen des brevets

(11) Veröffentlichungsnummer: **0 324 344**
**A1**

# (12) EUROPÄISCHE PATENTANMELDUNG

(21) Anmeldenummer: **89100026.7**

(22) Anmeldetag: **03.01.89**

(51) Int. Cl.4: **C07D 493/10 , B41M 5/12 , //(C07D493/10,311:00,307:00)**

(30) Priorität: **12.01.88 DE 3800575**

(43) Veröffentlichungstag der Anmeldung: **19.07.89 Patentblatt 89/29**

(84) Benannte Vertragsstaaten: **CH DE FR GB IT LI**

(71) Anmelder: **BASF Aktiengesellschaft**
**Carl-Bosch-Strasse 38**
**D-6700 Ludwigshafen(DE)**

(72) Erfinder: **Mayer, Udo, Dr.**
**Max-Slevogt-Strasse 27**
**D-6710 Frankenthal(DE)**
Erfinder: **Landmann, Bernd, Dr.**
**Budapester Strasse 45**
**D-6700 Ludwigshafen(DE)**

(54) **Fluoranverbindungen.**

(57) Fluoranverbindungen der allgemeinen Formel (I)

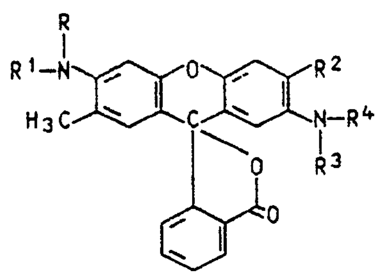

(I),

in der
R für Methyl oder Ethyl,
$R^1$ für Wasserstoff, $C_1$- bis $C_6$-Alkyl, Cyanethyl oder Benzyl,
$R^2$ für Wasserstoff oder Methyl,
$R^3$ für Wasserstoff, Methyl, Ethyl oder Cyanethyl und
$R^4$ für gegebenenfalls durch Chlor, Methyl und/oder $C_1$- bis $C_4$-Alkoxy ein-oder zweifach substituiertes Phenyl stehen, wobei $R^4$ nicht 2-Chlorphenyl sein kann, wenn $R^1$ und $R^2$ Wasserstoff sind.

Die Fluorane eignen sich hervorragend für druck- oder wärmeempfindliche Aufzeichnungsmaterialien. Die Fluorane (I) färben Streichrohpapier bzw. den Hintergrund nur in geringem Maße an. Die Färbungen sind lichtechter als die der bekannten Fluorane.

EP 0 324 344 A1

## Fluoranverbindungen

Aus der JP-A-180 557/1983 ist das Fluroranderivat der Formel (II)

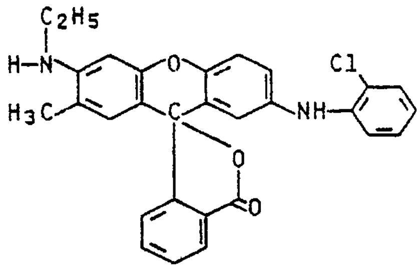

(II)

bekannt.

Weiterhin wird in der JP-A-59 115/1975 das Fluoranderivat der Formel (III)

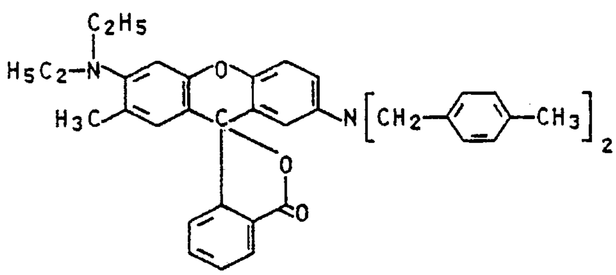

(III)

beschrieben.

Beide Fluorane weisen jedoch bei der Anwendung als Farbbildner in einem druckempfindlichen oder wärmeempfindlichen Aufzeichnungsmaterial schwerwiegende Mängel auf. Die Fluoranverbindung (II) gibt eine starke Streichrohpapier- bzw. Hintergrundanfärbung. Die Fluoranverbindung (III) weist nur eine geringe Lichtechtheit auf.

Aufgabe der vorliegenden Erfindung war es, neue Fluorane bereitzustellen, die im Vergleich zu den Verbindungen des Standes der Technik eine geringere Streichrohpapier- bzw. Hintergrundanfärbung liefern. Außerdem sollten die Fluorane eine höhere Lichtechtheit aufweisen als die bekannten.

Die Erfindung betrifft Fluoranverbindungen der allgemeinen Formel

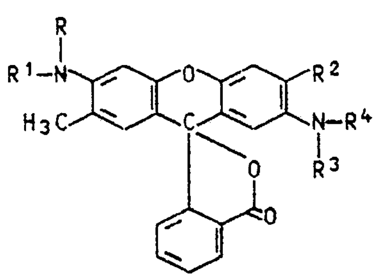

(I),

in der

R für Methyl oder Ethyl,

$R^1$ für Wasserstoff, $C_1$- bis $C_6$-Alkyl, Cyanethyl oder Benzyl,

$R^2$ für Wasserstoff oder Methyl,

$R^3$ für Wasserstoff, Methyl, Ethyl oder Cyanethyl und

$R^4$ für gegebenenfalls durch Chlor, Methyl und/oder $C_1$- bis $C_4$-Alkoxy ein-oder zweifach substituiertes Phenyl stehen, wobei $R^4$ nicht 2-Chlorphenyl sein kann, wenn $R^1$ und $R^2$ Wasserstoff sind.

Die erfindungsgemäßen Fluorane zeichnen sich gegenüber bekannten Fluoranverbindungen durch eine

geringere Streichrohpapier- bzw. Hintergrundanfärbung sowie durch eine höhere Lichtechtheit bei der Anwendung in druck- oder wärmeempfindlichen Aufzeichnungsmaterialien aus.

R steht für Methyl oder Ethyl, wobei Ethyl besonders bevorzugt ist.

$R^1$ steht für Wasserstoff, für $C_1$- bis $C_6$-Alkyl, Cyanethyl oder Benzyl. Wenn $R^1$ eine Alkylgruppe ist, kann diese linear oder verzweigt sein. Beispiele für solche Alkylgruppen sind Methyl, Ethyl, n-Propyl, Isopropyl, n-Butyl, Isobutyl, sek.-Butyl, Amyl, Isoamyl, n-Hexyl. Bevorzugt ist $R^1$ Wasserstoff und besonders bevorzugt Methyl.

$R^4$ steht für gegebenenfalls durch Chlor, Methyl und/oder $C_1$- bis $C_4$-Alkoxy ein- oder zweifach substituiertes Phenyl. $R^4$ umfaßt zum Beispiel Phenyl, 2-Chlorphenyl, 3-Chlorphenyl, 4-Chlorphenyl, 2-Methylphenyl, 3-Methylphenyl, 4-Methylphenyl, 2-Methoxyphenyl, 3-Methoxyphenyl, 4-Methoxyphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl. $R^4$ steht vorzugsweise für 2-Chlorphenyl oder 3-Chlorphenyl, insbesondere für Phenyl oder 4-Methylphenyl.

Die erfindungsgemäßen Fluoranverbindungen werden in an sich bekannter Weise durch Kondensation einer Benzoylbenzoesäure der allgemeinen Formel (IV)

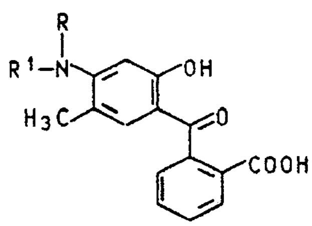

(IV),

in der R und $R^1$ die oben angegebene Bedeutung haben, mit einem Diphenylaminderivat der allgemeinen Formel (V)

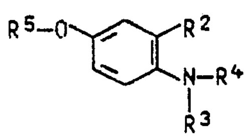

(V),

hergestellt, in der $R^2$, $R^3$ und $R^4$ die oben angegebene Bedeutung haben und $R^5$ für Wasserstoff, Methyl oder Ethyl steht. Als Kondensationsmedium dient Schwefelsäure der Konzentration 70 bis 100 %, vorzugsweise 85 bis 98 %. Die Kondensation wird bei einer Temperatur im Bereich von 0 bis 60 °C, vorzugsweise bei einer Temperatur von 5 bis 40 °C durchgeführt.

Die benötigten Benzoylbenzoesäuren IV werden durch Kondensation in Gegenwart von Mineralsäure von 3-Amino-1-oxo-isoindolenin mit 3-Amino-phenolen der allgemeinen Formel VI

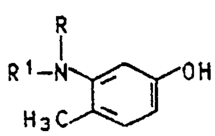

(VI),

in der R und $R^1$ die oben angegebene Bedeutung haben, und anschließende Verseifung hergestellt (Patent-Anmeldung ; O.Z. 0050/39676).

Die erfindungsgemäßen Fluorane sind farblos bis sehr schwach gefärbt. Sie ergeben im Kontakt mit sauren Entwicklern intensive braune, grüne oder schwarze Farbtöne und sind als Farbbildner für druck- oder wärmeempfindliche Aufzeichnungsmaterialien geeignet.

Die erfindungsgemäßen Fluoranverbindungen sind besonders für Durchschreibepapiere, bei denen die CF-Schicht Tone als Nehmer enthält, sowie für den Thermodruck geeignet.

Die Erfindung soll durch die folgenden Beispiele zusätzlich erläutert werden. Die in den Beispielen angegebenen Teile und Prozentangaben beziehen sich auf das Gewicht.

Beispiel 1

75,3 Teile 2-(4′-Ethylamino-2′-hydroxy-5′-methylbenzoyl)-benzoesäure werden bei 0 bis 5 °C in 500 Teilen 96 %iger Schwefelsäure gelöst. Anschließend werden bei der gleichen Temperatur 53,3 Teile 4-Methoxy-2-methyl-diphenylamin eingetragen. Die Reaktionsmischung wird 4 Stunden lang bei 0 °C und 48 Stunden lang bei Raumtemperatur gerührt und dann auf 1000 Teile Eis gegossen. Der ausgefallene Feststoff wird abgesaugt und mit Wasser gewaschen. Der Preßkuchen wird mit 415 Teilen 10 %iger Natronlauge und 325 Teilen Toluol 1 Stunde lang auf 110 °C erhitzt. Die Phasen werden heiß getrennt. Die organische Phase wird mit 200 Teilen heißem Wasser gewaschen und auf ein Drittel des Volumens eingeengt. Nach dem Abkühlen wird der ausgefallene Feststoff abgesaugt und mit Toluol gewaschen. Man erhält 86,9 Teile 3,7-Dimethyl-6-ethylamino-2-phenylaminofluoran in Form blaßrosa gefärbter Kristalle; Schmelzpunkt: 238-240 °C.

Die Lösung des Farbbildners in Toluol liefert auf einem mit Ton beschichteten Nehmerpapier eine braune Färbung.

Beispiel 2

15,7 Teile 2-(4′-(N-Ethyl-N-methylamino)-2′-hydroxy-5′-methylbenzoyl)-benzoesäure werden bei 0 °C in 200 Teilen 96 %iger Schwefelsäure gelöst. Anschließend werden 11,7 Teile 2-Chlor-4′-methoxy-diphenylamin innerhalb von 15 Minuten eingetragen. Die Reaktionsmischung wird 48 Stunden lang bei 25 °C gerührt und analog Beispiel 1 aufgearbeitet. Man erhält 8,5 Teile 6-(N-Ethyl-N-methylamino)-2-(2′-chlorphenylamino)-7-methylfluoran in Form blaßrosa gefärbter Kristalle. Schmelzpunkt: 177 bis 181 °C.

Eine Toluollösung des Farbbildners gibt auf einem mit Ton beschichteten Nehmerpapier eine violettstichig-schwarze Färbung.

Beispiel 3

15,7 Teile 2-(4′-(N-Ethyl-N-methylamino)-2′-hydroxy-5′-methylbenzoyl)-benzoesäure werden bei 0 °C in 200 Teilen 96 %iger Schwefelsäure gelöst. Anschließend werden 11,7 Teile 3-Chlor-4′-methoxy-diphenylamin innerhalb von 15 Minuten eingetragen. Die Reaktionsmischung wird 48 Stunden lang bei 25 °C gerührt und analog Beispiel 1 aufgearbeitet. Man erhält 5,6 Teile 6-(N-Ethyl-N-methylamino)-2-(3′-chlorphenylamino)-7-methylfluoran in Form blaßrosa gefärbter Kristalle vom Schmelzpunkt 169 bis 172 °C.

Auf mit Ton beschichtetem Nehmerpapier erhält man eine violettstichig-schwarze Färbung.

Beispiel 4

15,7 Teile 2-(4′-(N-Ethyl-N-methylamino)-2′-hydroxy-5′-methylbenzoyl)-benzoesäure werden bei 0 °C in 200 Teilen 96 %iger Schwefelsäure gelöst. Anschließend werden 10,0 Teile 4-Methoxy-diphenylamin innerhalb von 15 Minuten eingetragen. Die Reaktionsmischung wird 48 Stunden lang bei 25 °C gerührt und analog Beispiel 1 aufgearbeitet. Man erhält 7,0 Teile 6-(N-Ethyl-N-methylamino)-7-methyl-2-phenylaminofluoran in Form blaßrosa gefärbter Kristalle vom Schmelzpunkt 177 bis 180 °C.

Auf mit Ton beschichtetem Nehmerpapier erhält man eine trübe grüne Färbung.

Beispiel 5

15,7 Teile 2-(4′-(N-Ethyl-N-methylamino)-2′-hydroxy-5′-methylbenzoyl)-benzoesäure werden bei 0 °C in 200 Teilen 96 %iger Schwefelsäure gelöst. Anschließend werden 10,7 Teile 4-Methoxy-4′-methyl-diphenylamin innerhalb von 15 Minuten eingetragen. Die Reaktionsmischung wird 48 Stunden lang bei 25 °C gerührt und analog Beispiel 1 aufgearbeitet. Man erhält 8,8 Teile 6-(N-Ethyl-N-methylamino)-7-methyl-2-(4′-methylphenylamino)-fluoran in Form blaßrosa gefärbter Kristalle vom Schmelzpunkt 224 bis 228 °C.

Auf mit Ton beschichtetem Nehmerpapier erhält man eine trübe grüne Färbung.

Beispiel 6

15,7 Teile 2-(4′-(N-Ethyl-N-methylamino)-2′-hydroxy-5′-methylbenzoyl)-benzoesäure werden bei 0 °C in 200 Teilen 96 %iger Schwefelsäure gelöst. Anschließend werden 10,7 Teile 4-Methoxy-N-methyl-diphenylamin innerhalb von 15 Minuten eingetragen. Die Reaktionsmischung wird 48 Stunden lang bei 25 °C gerührt und analog Beispiel 1 aufgearbeitet. Man erhält 7,0 Teile 6-(N- Ethyl-N-methylamino)-7-methyl-2- (N-methyl-N-phenylamino)-flouran in Form Blaßrosa gefärbter Kristalle vom Schmelzpunkt 120 °C

Auf mit Ton beschichtetem Nehmerpapier erhält man eine grüne Färbung.

Beispiel 7

15,7 Teile 2-(4′-(N-Ethyl-N-methylamino)-2′-hdroxy-5′-methylbenzoyl)-benzoesäure werden bei 0 °C in 200 Teilen 96 %iger Schwefelsäure gelöst. Anschließend werden 11,4 Teile 4-Methoxy-4′-methyl-N-methyl-diphenylamin innerhalb von 15 Minuten eingetragen. Die Reaktionsmischung wird 48 Stunden lang bei 25 °C gerührt und analog Beispiel 1 aufgearbeitet. Man erhält 7,4 Teile 6-(N-Ethyl-N-methylamino)-7-methyl-2-(N-methyl-4′-methylphenylamino)-fluoran in Form blaßrosa gefärbter Kristalle vom Schmelzpunkt 140 bis 143 °C.

Der Farbbildner liefert auf mit Ton beschichtetem Nehmerpapier eine grüne Färbung.

Beispiel 8

15,7 Teile 2-(4′-(N-Ethyl-N-methylamino)-2′-hydroxy-5′-methylbenzoyl)-benzoesäure werden bei 0 °C in 150 Teilen 96 %iger Schwefelsäure gelöst. Anschließend werden 10,7 Teile 4-Methoxy-2-methyl-diphenylamin innerhalb von 15 Minuten eingetragen. Die Reaktionsmischung wird 6 Stunden lang bei 25 °C gerührt und analog Beispiel 1 aufgearbeitet. Man erhält 13,3 Teile 3,7-Dimethyl-6-(N-ethyl-N-methylamino)-2-phenylaminofluoran in Form blaßbeige gefärbter Kristalle vom Schmelzpunkt 202 bis 205 °C.

Auf mit Ton beschichtetem Nehmerpapier erhält man eine schwarze Färbung.

Beispiel 9

89,8 Teile 2-(4′-Dimethylamino-2′-hydroxy-5′-methylbenzoyl)-benzoesäure werden bei 0 °C in 900 Teilen 96 %iger Schwefelsäure gelöst. Anschließend werden bei der gleichen Temperatur 63,9 Teile 4-Methoxy-2-methyl-diphenylamin eingetragen. Die Reaktionsmischung wird 4 Stunden bei 0 °C und 48 Stunden bei 25 °C gerührt und analog Beispiel 1 aufgearbeitet. Man erhält 100,6 Teile 3,7-Dimethyl-6-dimethylamino-2-phenylaminofluoran in Form blaßbeigefarbener Kristalle vom Schmelzpunkt 185 - 188 °C.

Auf mit Ton beschichtetem Nehmerpapier erhält man eine schwarze Färbund.

**Ansprüche**

1. Fluoranverbindungen der allgemeinen Formel (I)

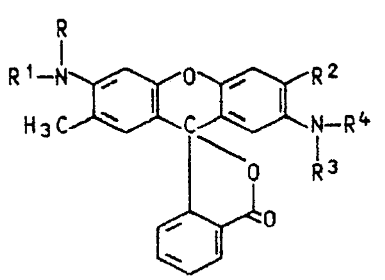

(I),

in der

R für Methyl oder Ethyl,

$R^1$ für Wasserstoff, $C_1$- bis $C_6$-Alkyl, Cyanethyl oder Benzyl,

$R^2$ für Wasserstoff oder Methyl,

$R^3$ für Wasserstoff, Methyl, Ethyl oder Cyanethyl und

$R^4$ für gegebenenfalls durch Chlor, Methyl und/oder $C_1$- bis $C_4$-Alkoxy ein- oder zweifach substituiertes Phenyl stehen, wobei $R^4$ nicht 2-Chlorphenyl sein kann, wenn $R^1$ und $R^2$ Wasserstoff sind.

2. Fluorane gemäß Anspruch 1, dadurch gekennzeichnet, daß R für Ethyl steht.

3. Fluorane gemäß Anspruch 1 oder 2, dadurch gekennzeichnet, daß $R^1$ für $C_1$- bis $C_6$-Alkyl, Cyanethyl oder Benzyl steht.

4. Fluorane gemäß Anspruch 1 oder 2, dadurch gekennzeichnet, daß $R^1$ für Methyl steht.

5. Fluorane gemäß Anspruch 1, 2, 3 oder 4, dadurch gekennzeichnet, daß $R^3$ für Wasserstoff oder Methyl steht.

6. Fluorane gemäß den Ansprüchen 1, 2, 3, 4 oder 5, dadurch gekennzeichnet, daß $R^4$ für Phenyl, 2- oder 3-Chlorphenyl oder 4-Methylphenyl steht.

7. Fluorane gemäß Anspruch 1, 2, 3, 4, oder 5, dadurch gekennzeichnet, daß $R^4$ für Phenyl oder 4-Methylphenyl steht.

8. Verwendung der Fluorane gemäß den Ansprüchen 1 bis 7 als Farbbildner in druckempfindlichen oder wärmeempfindlichen Aufzeichnungsmaterialien.

Europäisches Patentamt

# EUROPÄISCHER RECHERCHENBERICHT

Nummer der Anmeldung

EP 89 10 0026

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| --- | --- | --- | --- |
| Y | PATENT ABSTRACTS OF JAPAN Band 8, Nr. 15, 21. Januar 1984 (C-206)(1452); & JP - A - 58 180 557 (RICOH K.K.) 22.10.1983 (Kat. D,Y) | 1-3,5,8 | C 07 D 493/10<br>B 41 M 5/12 //<br>(C 07 D 493/10<br>C 07 D 311:00<br>C 07 D 307:00 ) |
| Y | DE-A-3 407 369 (SHIN NISSO KAKO CO. LTD) * Ansprüche 1-3 * | 1-3,5,8 | |
| Y | DE-A-2 245 504 (NISSO KAKO CO. LTD.) * Ansprüche 1,2,6,7 * | 1-3,5,8 | |
| A | CHEMICAL ABSTRACTS Band 84, Nr. 1, 5. Januar 1976, Seite 411, Abstract 10899y; & JP - A - 75 59115 (Kat. D,A) | 1,8 | |
| A | EP-A-0 138 177 (BASF AG) * Zusammenfassung; Beispiele * | 1,8 | |

RECHERCHIERTE SACHGEBIETE (Int. Cl.4)

C 07 D 493/00

Der vorliegende Recherchenbericht wurde für alle Patentansprüche erstellt

| Recherchenort | Abschlußdatum der Recherche | Prüfer |
| --- | --- | --- |
| BERLIN | 20-03-1989 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)